(19) Europäisches Patentamt
European Patent Office
Office européen des brevets

(11) Publication number: **0 319 644**
**A2**

# (12) EUROPEAN PATENT APPLICATION

(21) Application number: **88110388.1**

(22) Date of filing: **29.06.88**

(51) Int. Cl.4: **C07C 149/24 , C07D 211/20 , C07C 147/14 , A61K 31/16 , A61K 31/445**

Amended claim in accordance with Rule 86 (2) EPC for the following Contracting State: ES

(30) Priority: **09.11.87 JP 282747/87**

(43) Date of publication of application:
**14.06.89 Bulletin 89/24**

(84) Designated Contracting States:
**BE CH DE ES FR GB IT LI NL SE**

(71) Applicant: **GREEN CROSS CORPORATION**
**15-1, Imabashi 1-chome Higashi-ku**
**Osaka-shi**
**Osaka(JP)**

(72) Inventor: **Iwai, Masakazu Forumu**
**Momogaike Koen 203 1-12-18,**
**Mimogaike-cho**
**Abeno-ku Osaka-shi Osaka(JP)**
Inventor: **Kohda, Isao**
**7-15-1-405, Minami Mukonoso**
**Amagasaki-shi Hyogo(JP)**
Inventor: **Fukaya, Chikara**
**2-11-33-604, Kema-cho Miyokojima-ku**
**Osaka-shi Osaka(JP)**
Inventor: **Arakawa, Yoshio**
**7-81, Aoyama**
**Nara-shi Nara(JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

## (54) Alkylated amide derivatives, their use and pharmaceutical compositions containing them.

(57) An alkylated amide derivative prepared by amide coupling of sulfur-containing amine and an unsaturated fatty acid with 4 to 18 carbon atoms, wherein the SH group of the sulfur-containing amine portion is alkylated by a group selected from an alkyl group, a cycloalkyl-alkyl group, an aralkyl group, an N-alkyl-piperidinyl-alkyl group, a piperidinyl-alkyl group, and an N,N-dialkylcarboxamide-alkyl group, which in form of pharmaceutical compositions has excellent activities against peptic ulcer in mammals and is useful for the making of an antiulcer agent.

EP 0 319 644 A2

# ALKYLATED AMIDE DERIVATIVES, THEIR USE AND PHARMACEUTICAL COMPOSITIONS CONTAINING THEM

## FIELD OF THE INVENTION

The present invention relates to alkylated amide derivative of a sulfur-containing amine and an unsaturated fatty acid.

## BACKGROUND OF THE INVENTION

The alkylated H-chain and L-chain of human serum IgG are both known to have potent inhibitory activity against peptic ulcer and inflammation. One of the mechanisms of this action, the role of potent inhibition of gastric juice secretion by these substances in cases of peptic ulcer, has been described. As for the antiinflammatory activities, inhibition of vascular permeability and leukocytoplania have been reported. The -SH group in the hinge region of IgG has been suggested as the active moiety.

Based on this knowledge, the present applicant discovered that the amide derivatives prepared by amide coupling of a sulfur-containing amine and an unsaturated fatty acid with 4 to 18 carbon atoms possess excellent activity against peptic ulcer and inflammation (EP-A-0 195 433).

## SUMMARY OF THE INVENTION

It is a primary object of this invention to present compounds possessing more potent activity against peptic ulcer than those disclosed in the preceding European patent publication.

The present inventors carried out studies for achieving the above object, and found that specific compounds with in the scope of the disclosure in EP-A-0 195 433 but not specifically described therein can achieve the intended object, and further continued studies to complete the present invention.

That is, the present invention presents alkylated amide derivatives prepared by amide coupling of an amino group of a sulfur-containing amine (an amino compound possessing sulfur atoms) and a carboxyl group of an unsaturated fatty acid with 4 to 18 carbon atoms, wherein the SH group of the sulfur-containing amine is alkylated by a group selected from among an alkyl group, a cycloalkyl-alkyl group, an araklyl group, an N-alkyl-piperdinyl-alkyl group, a piperidinyl-alkyl group and an N,N-dialkyl-carboxamide-alkyl group.

## DETAILED DESCRIPTION OF THE INVENTION

In this invention, examples of the group for alkylating the SH group of the sulfur-containing amine may include an alkyl group, a cyclo alkyl-alkyl group, an araklyl group, an N-alkyl-piperidinyl-alkyl group, a piperidinyl-alkyl group, and an N,N-dialkyl-carboxamide-alkyl group.

As said alkyl group or -alkyl (including -alkyl in -dialkyl) portion, those with either a straight chain or branched form with 1 to 4 carbon atoms are preferable. Practical examples of alkylating groups include the following.

1) Alkyl group:

Methyl, ethyl, n-propyl, n-butyl and others with 1 to 4 carbon atoms.

2) Cycloalkyl-alkyl group:

Cyclohexylmethyl, cyclohexylethyl, etc.

3) Aralkyl group:

Benzyl, phenylethyl, etc.

4) N-alkyl-piperidinyl-alkyl group:

N-methyl-2-piperidinyl-methyl, etc.

5) Piperidinyl-alkyl group:

β-(1-piperidinyl)-ethyl, etc.

6) N,N-dialkyl carboxamide-alkyl group:

N,N-dimethyl-carboxamide methyl, N,N-diethyl-carboxamide methyl, etc.

These groups are bound to the sulfur atom in the sulfur-containing amine through the alkyl portion as underlined above.

Among these groups, a cycloalkyl-alkyl group is particularly preferred.

In this invention, the sulfur-containing amine need not be specifically defined nor restricted except as far as it is an amino compound possessing sulfur atoms. The number of amino groups in the sulfur-containing amine preferably should be 1 or 2. The SH group of the sulfur-containing amine may be replaced preliminarily by said specific alkyl group, so that an alkylated amide derivative prepared by coupling said alkylated sulfur-containing amine and said unsaturated fatty acid need not be alkylated in a later process. However, an amide derivative prepared by coupling nonalkylated sulfur-containing amine and said unsaturated fatty acid must be alkylated in a later process.

Practical examples of sulfur-containing amines may include cysteine, cystamine, cysteamine, taurine, lauthionine, aminopropanethiole, methionine, and ethionine, with cysteamine being preferred. As the alkylated sulfur-containing amine, compounds prepared by alkylating the SH group of cysteine, cysteamine, or aminopropanethiole or methionine, ethionine and others are preferable, although alkylated cysteamine is most desirable.

The S-oxide derivatives of the sulfur-containing amides can be prepared using conventional technique and used as the active biological agent.

The unsaturated fatty acid is not specifically limited except that it contains 4 to 18 carbon atoms, or preferably 8 to 13. As the unsaturated group, a double bond is preferred, and the number of unsaturated groups is not limited specifically, but preferably should be 1 to 3. The position of the unsaturated group is not limited specifically. Practical examples of unsaturated fatty acid include the following.

2 - Octenoic acid;
$CH_3(CH_2)_4CH=CHCOOH$
2 - Decenoic acid; $CH_3(CH_2)_6CH=CHCOOH$
2 - Undecenoic acid;
$CH_3(CH_2)_7CH=CHCOOH$
2 - Tridecenoic acid;
$CH_3(CH_2)_9CH=CHCOOH$
Sorbic acid;
$CH_3CH=CHCH=CHCOOH$

As such unsaturated fatty acids, in particular, the unsaturated fatty acid expressed by the formula:
$CH_3(CH_2)_mCH=CH(CH_2)_nCOOH$
(where m + n = an integer from 0 to 14) is most preferred. The sum of m + n denotes an integer from 0 to 14, preferably 4 to 9, and more preferably 6 or 7. More specifically, m is preferably an integer from 0 to 14, more preferably 4 to 9, and most preferably 6 or 7, while n is preferably 0 to 1, or more preferably 0.

As a preferred embodiment of the amide derivative, there is a compound expressed by the formula:

$CH_3(CH_2)_mCH = CH(CH_2)_nCONH\text{-}A\text{-}Y\text{-}X$ (I)
wherein m and n have the same meaning as above, A is an alkylene group, Y represents a sulfur atom or a sulfinyl group, and X represents a group for alkylating said SH group, that is, a group selected from among an alkyl group, a cycloalkyl-alkyl group, an aralkyl group, an N-alkyl-piperidinyl-alkyl group, a piperidinyl-alkyl group and an N,N-dialkyl-carboxamide-alkyl group.
As the alkylene group expressed by A in formula (I), those in straight chain or branched form having 1 to 3 carbon atoms may be used. Practically, methylene and ethylene are understood, and in particular ethylene is preferable.

The amide derivatives of this invention may be prepared by a known amidation process. For example, an unsaturated fatty acid or its reactive derivative is caused to react with a sulfur-containing amine in the presence of, if necessary, a dehydrating agent, and is alkylated as required.

As the reactive derivative of the unsaturated fatty acid, acid halides such as acid chloride and acid bromide, active ester, acid anhydride and others may be used.

The reactive derivative of the unsaturated fatty acid, for example, acid halide is manufactured usually by treating the unsaturated fatty acid with phosphorus trichloride, phosphorus pentachloride or more preferably thionyl chloride (mixing ratio by weight, fatty acid : thionyl chloride = 1 : 1 to 5). At this time, the reaction should last for 4 to 29 hours at 40 to 80° C. This acid halide is refined, for example, by distillation.

Examples of dehydrating agents may include N,N′-di-substitutional carbodiimides such as N,N′-dicyclohexylcarbodiimide, or N,N′-carbonyldiimidazole, or the like.

This amidation reaction is conducted in the presence of a solvent inactive to the reaction. Usable solvents may be, for example, ethyl acetate, chloroform, and ether. The reaction time is usually 30 minutes to 4 hours, and the reaction temperature is generally 0 to 50° C, approximately.

About 1 to 3 mols of unsaturated fatty acid is used per 1 mol of sulfur-containing amine. The dehydrating agent should be blended therewith preferably in equal mole to unsaturated fatty acid used.

In the case of an amide derivative possessing a free SH group prepared by using a sulfur-containing amide which is not alkylated, the amidation product is further alkylated to obtain an alkylated amide derivative. If the amide derivative possesses the S-S (disulfide) group, it should be alkylated after reducing the S-S group into an SH group, so that an alkylated amide derivative may be obtained. This alkylation is conducted by a known means.

Specifically, after reaction with trialkylphosphine (for example, tributylphosphine) for 10 minutes to 2 hours at 10 to 30° C in a mixed solution of alcohol (e.g. methanol) and water, the reaction is carried out for 5 to 20 hours at 0 to 30° C together with a halogenated alkyl or substituted alkyl in an alkaline condition, for example, in the presence of sodium hydroxide, potassium hydroxide.

Then, the synthesized alkylated amide derivative may be isolated and purified by a known means such as solution inversion, recrystallization and chromatography.

The alkylated amide derivatives of this invention possess excellent activities against peptic ulcer in mammals (humans, horses, dogs, mice, rats, etc.), and are useful as antiulcer agents.

The alkylated amide derivatives of this invention may be used either orally or nonorally in the form of a pharmaceutical preparation together with proper and pharmaceutically permitted carriers.

The pharmaceutical preparation may be in the form of tablets, capsules, powders, suppositories, injections or other common types of pharmaceutical preparations.

In the oral route, for example, the alkylated amide derivatives of this invention may be administered by a single dose of 50 to 250 mg once to several times a day, but the dose will be different from individual to individual depending on the age and body weight of the subjects and/or the severity of the disease to be treated or the reaction to the treatment.

The present invention will be illustrated in more detail by the following examples, but these examples are only illustrative and not restrictive.

## EXAMPLE 1

Synthesis of S-(cyclohexylethyl)-N-(2-decenoyl) cysteamine (Compound 2)

Compound 1 (1.5 g) obtained in Reference Example 1 was suspended in 38 ml of methanol-water (9:1), and tri-n-butylphosphine (0.9 ml) was dripped in under a nitrogen gas atmosphere, and the reaction was conducted for 1 hour at room temperature. To this reaction solution, (2-iodoethyl) cyclohexane (1.64 g) was

added, and the mixture was cooled, to which 1 N sodium hydroxide (6.9 ml) was dripped in. After this addition, the solution was stirred overnight at room temperature. This reaction solution was concentrated at reduced pressure, and to the obtained concentrate, ethyl acetate and water were added, the mixture was shaken, and the organic solvent layer was separated off. After drying the organic solvent layer, the solvent was distilled off, and the obtained residue was refined by column chromatography using silica gel, and an oily Compound 2 was obtained (1.88 g, 84%).

(1) IR $\nu_{max}^{Next}$ $cm^{-1}$ : 3270, 2900, 2840, 1665, 1630, 1540, 1450, 970
(2) NMR [$CDCl_3$) δ ppm :
0.88 (brt, 3H), 2.10-2.23 (m, 2H),
2.53 (t, 2H, J=7.0Hz),
2.69 (t, 2H, J=6.5Hz),
3.51 (q, 2H, J=6.5Hz),
5.77 (dt, 1H, J=15.3, 1.4Hz),
5.92 (br. 1H),
6.84 (dt, 1H, J=15.3, 7.0Hz).

## EXAMPLE 2

Synthesis of S-(cyclohexylmethyl)-N-(2-decenoyl) cysteamine (Compound 3)

Using iodomethyl cyclohexane, reduction and alkylation were conducted by the same operation as in Example 1, and an oily Compound 3 (76%) was obtained.

The properties of Compound 3 were as follows.

(1) IR $\nu_{max}^{Nujol}$ $cm^{-1}$ : 3400, 2920, 2840, 1665, 1620, 1540, 980
(2) NMR ($CDCl_3$) δ ppm :
0.88 (brt, 3H), 2.10-2.23 (m, 2H),
2.42 (t, 2H, J=6.8Hz),
2.67 (t, 2H, J=6.5Hz),
3.50 (q, 2H, J=6.2Hz),
5.78 (dt, 1H, J=15.3, 1.4Hz),
5.97 (br. 1H),
6.84 (dt, 1H, J=15.3, 7.0Hz).

## EXAMPLE 3

Synthesis of S-methyl-N-(2-decenoyl) cysteamine (Compound 4)

Using iodomethyl, reduction and alkylation were conducted by the same operation as in Example 1, and Compound 4 (83%) was obtained.

The properties of Compound 4 were as follows.

(1) Melting point : 51.0 to 51.5 °C (n-hexane)
(2) IR $\nu_{max}^{KBr}$ $cm^{-1}$ : 3300, 3090, 2930, 2860, 1665, 1630, 1545, 980
(3) NMR ($CDCl_3$) δ ppm :
0.88 (brt, 3H), 2.12 (s, 3H),
2.10-2.22 (m, 2H),
2.67 (t, 2H, J=6.3Hz),
3.53 (q, 2H, J=6.3Hz),
5.79 (dt, 1H, J=15.3, 1.4Hz),
6.05 (br. 1H),
6.85 (dt, 1H, J=15.3, 7.0Hz).

## EXAMPLE 4

Synthesis of S-butyl-N-(2-decenoyl) cysteamine (Compound 5)

Using bromide n-butyl, reduction and alkylation were conducted in the same manner as in Example 1, and Compound 5 (87%) was obtained.

The properties of Compound 5 were as follows.

(1) Melting point: 48.0 to 48.5 °C (n-hexane)
(2) IR $\nu_{max}^{KBr}$ cm⁻¹: 3220, 3070, 2920, 2850, 1660, 1620, 1550, 1465, 975
(3) NMR ($CDCl_3$) δ ppm :
0.88 (brt, 3H), 0.91 (t, 3H, J = 7.0Hz),
2.10-2.20 (m, 2H),
2.53 (t, 2H, J = 7.0Hz),
2.69 (t, 2H, J = 6.3Hz),
3.51 (q, 2H, J = 6.3Hz),
5.78 (dt, 1H, J = 15.3, 1.4Hz).
5.95 (br, 1H),
6.85 (dt, 1H, J = 15.3, 7.0Hz).

## EXAMPLE 5

Synthesis of S-benzyl-N-(2-decenoyl) cysteamine (Compound 6)

Using benzyl bromide, reduction and alkylation were conducted by the same operation as in Example 1, and Compound 6 (90%) was obtained.

The properties of Compound 6 were as follows.

(1) Melting point : 52.0 to 52.5 °C (n-hexane-ethyl acetate)
(2) IR $\nu_{max}^{KBr}$ cm⁻¹ : 3300, 3080, 2920, 2860, 1665, 1625, 1545, 980, 700.
(3) NMR ($CDCl_3$) δ ppm :
0.88 (brt, 3H), 2.05-2.22 (m, 2H),
2.58 (t, 2H, J = 6.5Hz),
3.43 (q, 2H, J = 6.3Hz), 3.71 (s, 2H),
5.74 (dt, 1H, J = 15.3, 1.4Hz),
6.05 (br, 1H),
6.81 (dt, 1H, J = 15.3, 7.0Hz).
7.15-7.50 (m, 5H)

## EXAMPLE 6

Synthesis of S-(2-phenylethyl)-N-(2-decenoyl) cysteamine (Compound 7)

Using (2-bromoethyl)benzene, reduction and alkylation were conducted by the same operation as in Example 1, and Compound 7 (93%) was obtained.

The properties of Compound 7 were as follows.

(1) Melting point : 53.0 to 53.5 °C (n-hexane-ethyl acetate)
(2) IR $\nu_{max}^{KBr}$ cm⁻¹ : 3280, 3050, 2910, 2840, 1640, 1620, 1540, 980, 705.
(3) NMR ($CDCl_3$) δ ppm :
0.88 (brt, 3H), 2.10-2.23 (m, 2H),
2.69 (t, 2H, J = 6.4Hz),
2.73-2.93 (m, 4H),

3.49 (q, 2H, J=6.3Hz),
5.75 (dt, 1H, J=15.3, 1.4Hz),
5.89 (br, 1H),
6.83 (dt, 1H, J=15.3, 7.0Hz).
7.16-7.37 (m, 5H)

## EXAMPLE 7

Synthesis of N-(2-decenoyl)-S-[(1-methylpiperidyl)methyl] cysteamine (Compound 8)

Using 1-methyl-2-chloromethylpiperidine hydrochloride, reduction and alkylation were conducted by the same operation as in Example 1 (except that 1 N sodium hydroxide was used in a double volume), and an oily Compound 8 (97%) was obtained.

The properties of Compound 8 were as follows.

(1) IR $\nu_{max}^{Next}$ $cm^{-1}$ : 3300, 3090, 2920, 2870, 2800, 1665, 1630, 1540, 975.
(2) NMR ($CDCl_3$) δ ppm :
0.88 (brt, 3H), 2.32 and 2.43 (s, 3H),
3.52 (q, 2H, J=6.3Hz),
5.78 (dt, 1H, J=15.3, 1.4Hz),
6.13 (br, 1H),
6.84 (dt, 1H, J=15.3, 7.0Hz).

## EXAMPLE 8

Synthesis of S-(2-cyclohexylethyl)-N-(2-decenoyl) cysteamine S-oxide

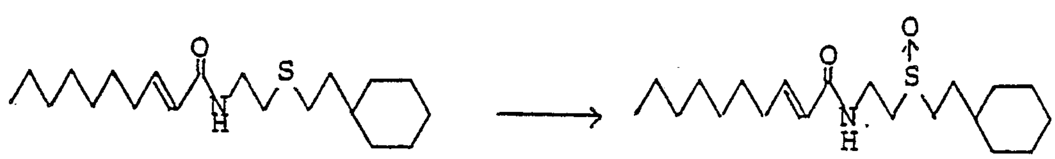

Compound 2 (640 mg) was dissolved in 20 ml of methanol and an aqueous solution containing 450 mg of sodium periodate (5 ml) was added thereto. After reaction for an hour at room temperature under stirring, 50 ml of water was added to the reaction mixture. The perecipitate formed was filtered off and washed with water. After drying, the precipitate was purified by recrystalization from ethyl acetate.

yield: 522 mg (88%), mp: 92-93 °C
(1) IR $\nu_{max}^{KBr}$ $cm^{-1}$: 3280, 2910, 2830, 1670, 1630, 1550, 1060
(2) NMR ($CDCl_3$) δ ppm:
0.88 (brt, 3H),
2.10-2.25 (m, 2H),
2.65-2.85 (m, 3H),
3.00-3.13 (m, 1H),
3.84 (q, J=5.7Hz, 2H),
5.78 (dt, J=15.3, 1.5HZ, 1H),
6.76 (brs, 1H), 6.83 (dt, J=15.3, 6.9Hz, 1H)

## EXAMPLE 9

Synthesis of S-butyl-N-(2-decenoyl) cysteamine S-oxide

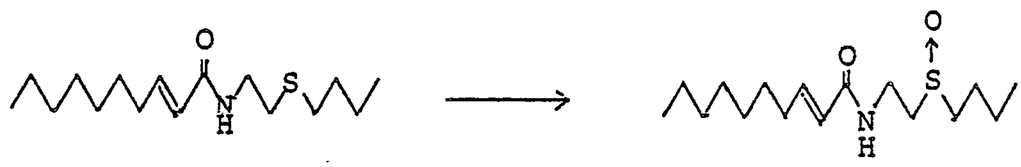

Using Compound 5, the above compound was prepared in the same manner as in Example 8. (85%)
$IR\nu_{max}^{KBr}$ $cm^{-1}$: 3220, 3070, 2920, 2840, 1660, 1620, 1550, 1060,

## EXAMPLE 10

Synthesis of S-(2-phenylethyl)-N-(2-decenoyl) cysteamine S-oxide

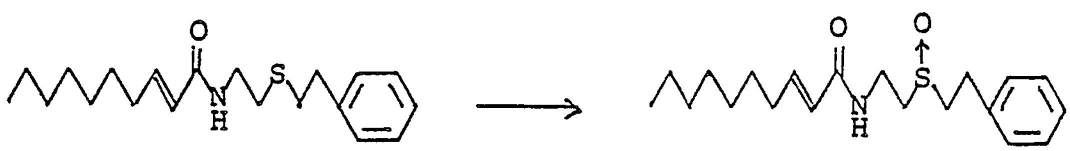

Using Compound 7, the above compound was prepared in the same manner as in Example 8. (80%)
$IR\nu_{max}^{KBr}$ $cm^{-1}$: 3280, 3050, 2910, 2840, 1640, 1640, 1060, 980, 705

## REFERENCE EXAMPLE 1

Synthesis of N,N′-bis(2-decenoyl) cysteamine (Compound 1)

Mixing 2-decenoic acid (15 g) and thionyl chloride (20 ml), the mixture was heated and stirred for 15 hours. After reaction, thionyl chloride was distilled off, the residue was distilled at reduced pressure, and an acid chloride was obtained (12.3 g, 74%, bp: 102.5 to 107.5°C). To a mixed solution of cystamine (4.2 g) and triethylamine (8.5 ml) with ethyl acetate (150 ml), ethyl acetate solution (15 ml) of said acid chloride (12.3 g) was dripped in under ice cooling. After reaction at 0°C for 30 minutes and thereafter overnight at room temperature, water (100 ml) was added to the reaction solution, and the solid matter was filtered out, and was further recrystallized by ethyl acetate, and Compound 1 was obtained (10.75 g, 75%).

Its properties were as follows.

(1) Melting point : 136 to 136.5°C
(2) IR $\nu_{max}^{KBr}$ $cm^{-1}$ : 3300, 3050, 2920, 2850, 1665, 1620, 1540, 975.
(3) NMR ($CDCl_3$ + $CF_3COOH$) $\delta$ ppm :
0.9 (br, 6H),
2.3 (br, 4H),
2.9 (t, 4H, J=7Hz),
3.8 (t, 4H, J=7Hz),
6.0 (d, 2H, J=15Hz),
7.1 (dt, 2H, J=15, 6Hz),
7.2 (br, 2H).

## REFERENCE EXAMPLE 2

Synthesis of S-(carboxamidemethyl)-N-(2-decenoyl) cysteamine (Compound 1′)

Compound 1 (2.0 g) was suspended in 60 ml of methanol-water (9:1) solution, and tributylphosphine (1.13 ml) was dripped in under a nitrogen atmosphere, and the reaction was conducted for 30 minutes at 0°C and 1 hour at room temperature. To this reaction solution, iodoacetamide (1.6 g) was added, the solution was cooled again, and 1 N sodium hydroxide (8.7 ml) was dripped in. After this addition, the solution was stirred at room temperature for 1 hour. To the reaction solution, water (100 ml) was added, and a white sediment was filtered out, and was recrystallized from ethanol-water, and Compound 1′ was obtained (2.1 g, 84%).

Its properties were as follows.

(1) Melting point : 151 to 151.5°C

(2) IR $\nu_{max}^{KBr}$ $cm^{-1}$ : 3370, 3300, 3170, 2920, 2850, 1650, 1620, 1540, 970

(3) NMR (DMSO-$d_6$) $\delta$ ppm :
0.86 (brt, 3H), 2.06-2.17 (m, 2H),
2.64 (t, 2H, J=6.4Hz),
3.09 (s, 2H), 3.30 (q, 2H, J=6.0),
5.87 (dt, 1H, J=15.4, 1.5Hz),
6.61 (dt, 1H, J=15.4, 7.0Hz),
7.04 (br, 1H), 7.45 (br, 1H),
8.06 (brt, 1H).

## TEST EXAMPLE 1

Each test compound was suspended in physiological saline containing 10% polyoxyethylene cured caster oil (HCO-60: manufactured by Nikko Chemicals), and was then tested.

The test was conducted according to the method of rat water immersed restraint stress ulcerization proposed by Okabe et al. (Jpn. J. Pharmacol., 18, 9, (1968)). That is, male Wistar rats weighing 230 to 250 g were fasted for 24 hours, put in a stress cage in the erect position, and were immersed in a thermostatic water tank at 22 ± 1°C up to the sternal process. The rats were sacrificed 7 hours later, and the stomach was removed. The pylorus was ligated and 10 ml of physiological saline was infused into the stomach. The cardia also was ligated and dipped in 1% formalin for 5 to 10 minutes. Afterwards, the stomach was opened along the greater curvature, and any blood deposited on the mucosa in the glandular portion was wiped off gently. The length of the remaining ulcers (erosions) was measured. The sum (mm) of the length of the ulcers in each rat was defined as the ulcer index. The test material was administered orally 90 minutes before stress loading (dose: 5 mg/kg).

The results are shown in Table 1.

Table 1

| Test Compound | Number of rats | Ulcer Index (Mean ± S.B.) | Inhibition rate (%) |
|---|---|---|---|
| Control (untreated) | 8 | 22.0 ± 3.6 | 0 |
| Compound 1' (Reference Example 2) | 8 | 12.8 ± 1.6 | 41.8 |
| Compound 2 | 7 | 6.7 ± 1.7 | 69.5 |
| Compound 8 | 8 | 8.0 ± 1.7 | 63.6 |

## TEST EXAMPLE 2

Male Wister rats weighing 200 to 220 g were fasted overnight and the preparation containing Compound 2 (S-(2-cyclohexylethyl)-N-(2-decenoyl) cysteamine) and 5% gum arabic or the preparation containing the S-oxide derivarive of Compound 2 (S-(2-cyclohexylethyl)-N-(2-decenoyl) cysteamine S-oxide) and 5% gum arabic was orally administered to each rat (2 ml/kg).

Antiulcer activity of each test compound was evaluated in the same manner as in Test Example 1.

The results are shown in Table 2.

Table 2

| Test Compound | Dose (mg/kg) | Number of rats | Ulcer Index (Mean ± S.B.) | Inhibition rate (%) |
|---|---|---|---|---|
| Control[a)] | 0 | 9 | 23.8 ± 4.8 | - |
| Compound 2 | 25 | 9 | 10.0 ± 1.5* | 58.0 |
| S-oxide of Compound 2 | 25 | 8 | 12.8 ± 1.9 | 46.2 |
| Note: | | | | |

a): Soybean oil
*: $p < 0.05$ (significantly different from the control group)

From the results shown in Table 2, it is found that both of compound 2 and the S-oxide derivative thereof exhibit an excellent antiulcer activity and no significant difference is observed between the two compounds.

While the Invention has been described in detail and with reference to specific embodiments thereof, it will be apparent to one skilled in the art that various changes and modifications can be made therein without departing from the spirit and scope thereof.

## Claims

1. An alkylated amide derivative prepared by amide coupling of sulfur-containing amine and an unsaturated fatty acid with 4 to 18 carbon atoms, wherein the SH group of the sulfur-containing amine portion is alkylated by a group selected from an alkyl group,
a cycloalkyl-alkyl group, an aralkyl group, an N-alkyl-piperidinyl-alkyl group, a piperidinyl-alkyl group, and an N,N-dialkylcarboxamide-alkyl group.

2. The alkylated amide derivative according to claim 1, wherein said sulfur-containing amine is alkylated cysteamine.

3. The alkylated amide derivative according to claim 1 or 2, wherein said unsaturated fatty acid is represented by the general formula:
$CH_3(CH_2)_mCH=CH(CH_2)_nCOOH$
wherein the sum of m and n is an integer ranging from 0 to 14.

4. The alkylated amide derivative according to claim 1 or 2 or 3, wherein said sulfur-containing amine is an S-oxide compound.

5. The alkylated amide derivative according to claim 1, wherein said group is selected from a methyl group, an ethyl group, an n-propyl group, an n-butyl group, a cyclohexylmethyl group, a cyclohexylethyl group, a benzyl group, a phenylethy group, an N-methyl-2-piperidinyl-methyl group, a $\beta$-(1-piperidinyl)ethyl group, an N,N-dimethylcarboxamide-methyl group and an N, N-diethylcarboxamide methyl group.

6. The alkylated amide derivative according to claim 1, wherein said unsaturated fatty acid is selected from 2-octenoic acid, 2-decenoic acid, 2-undecenoic acid, 2-tridecenoic acid and sorbic acid.

7. The alkylated amide derivative according to claim 1, which is selected from S-(cyclohexylethyl-N-(2-decenoyl) cysteamine, S-(cyclohexylmethyl)-N-(2-decenoyl) cysteamine, S-methyl-N-(2-decenoyl) cysteamine, S-butyl-N-(2-decenoyl) cysteamine, S-benzyl-N-(2-decenoyl) cysteamine, S-(2-phenylethyl)-N-(2-decenoyl) cysteamine, N-(2-decenoyl)-S-[(1-methylpiperidyl)methyl] cysteamine, S-(2-cyclohexylethyl)-N-(2-decenoyl) cysteamine S-oxide, S-butyl-N-(2-decenoyl) cysteamine S-oxide and S-(2-phenylethyl)-N-(2-decenoyl) cysteamine S-oxide.

8. A pharmaceutical composition comprising a pharmaceutical carrier and a derivative of claim 1.

9. The use of a derivative of claim 1 for the making of a medicament effective against peptic ulcer.

Claims for the following Contracting State: ES

1. A process for preparing an alkylated amide derivative which comprises conducting the amidation reaction between sulfur-containig amine and an unsaturated fatty acid with 4 to 18 carbon atoms, wherein the SH group of the sulfur-containing amine portion of the derivative prepared is alkylated by a group selected from an alkyl group, a cycloalkyl-alkyl group, an aralkyl group, an N-alkylpiperidinyl-alkyl group, a piperidinyl-alkyl group, and an N,N-dialkyl-carboxamide-alkyl group.

2. The process according to claim 1, wherein said sulfur-containing amine is alkylated cysteamine.

3. The process according to claim 1 or 2, wherein said unsaturated fatty acid is represented by the general formula:

$CH_3(CH_2)_mCH=(CH_2)_nCOOH$

wherein the sum of m and n is an integer ranging from 0 to 14.

4. The process according to claim 1 or 2 or 3, wherein said sulfur-containing amine is an S-oxide compound.

5. The process according to claim 1, wherein said group is selected from a methyl group, an ethyl group, an n-propyl group, an n-butyl group, a cyclohexylmethyl group, a cyclohexylethyl group, a benzyl group, a phenylethy group, an N-methyl-2-piperidinyl-methyl group, a $\beta$-(1-piperidinyl)ethyl group, an N,N-dimethylcarboxamide-methyl group and an N, N-diethylcarboxamide methyl group.

6. The process according to claim 1, wherein said unsaturated fatty acid is selected from 2-octenoic acid, 2-decenoic acid, 2-undecenoic acid, 2-tridecenoic acid and sorbic acid.

7. The process according to claim 1, wherein said alkylated amide derivative is selected from S-(cyclohexylethyl)-N-(2-decenoyl) cysteamine, S-(cyclohexylmethyl)-N-(2-decenoyl) cysteamine, S-methyl-N-(2-decenoyl) cysteamine, S-butyl-N-(2-decenoyl) cysteamine, S-benzyl-N-(2-decenoyl) cysteamine, S-(2-phenylethyl-N-(2-decenoyl) cysteamine, N-(2-decenoyl)-S-[(1-methylpiperidyl)methyl] cysteamine, S-(2-cyclohexylethyl)-N-(2-decenoyl) cysteamine S-oxide, S-butyl-N-(2-decenoyl) cysteamine S-oxide and S-(2-phenylethyl)-N-(2-decenoyl) cysteamine S-oxide.

8. The use of an alkylated amide derivative prepared by amide coupling of sulfur-containing amine and an unsaturated fatty acid with 4 to 18 carbon atoms, wherein the SH group of the sulfur-containing amine portion is alkylated by a group selected from an alkyl group, a cycloalkyl-alkyl group, an aralkyl group, an N- alkylpiperidinyl-alkyl group, a piperidinyl-alkyl group, and an N,N-dialkylcarboxamide-alkyl group for preparing a pharmaceutical composition for treating peptic ulcers.

9. The use according to claim 8, wherein said sulfur-containing amine is alkylated cysteamine.

10. The use according to claim 8, wherein said unsaturated fatty acid is represented by the general formula:

$CH_3(CH_2)_mCH=(CH_2)_nCOOH$

wherein the sum of m and n is an integer ranging from 0 to 14.

11. The use according to claim 8, wherein said sulfur-containing amine is an S-oxide compound.

12. The use according to claim 3, wherein said group is selected from a methyl group, an ethyl group, an n-propyl group, an n-butyl group, a cyclohexylmethyl group, a cyclohexylethyl group, a benzyl group, a phenylethy group, an N-methyl-2-piperidinyl-methyl group, a $\beta$-(1-piperidinyl)ethyl group, an N,N-dimethylcarboxamide-methyl group and an N, N-diethylcarboxamide methyl group.

13. The use according to claim 8, wherein said unsaturated fatty acid is selected from 2-octenoic acid, 2-decenoic acid, 2-undecenoic acid, 2-tridecenoic acid and sorbic acid.

14. The use according to claim 8, wherein said alkylated amide derivative is selected from S-(cyclohexylethyl)-N-(2-decenoyl) cysteamine, S-(cyclohexylmethyl)-N-(2-decenoyl) cysteamine, S-methyl-N-(2-decenoyl) cysteamine, S-butyl-N-(2-decenoyl) cysteamine, S-benzyl-N-(2-decenoyl) cysteamine, S-(2-phenylethyl)-N-(2-decenoyl) cysteamine, N-(2-decenoyl)-S-[(1-methylpiperidyl)methyl] cysteamine, S-(2-cyclohexylethyl)-N-(2-decenoyl) cysteamine S-oxide, S-butyl-N-(2-decenoyl) cysteamine S-oxide and S-(2-phenylethyl)-N-(2-decenoyl) cysteamine S-oxide.